# EUROPEAN PATENT APPLICATION

(11) **EP 2 127 684 A1**
(43) Date of publication of application: **02.12.2009**
(21) Application number: 08156565.7
(22) Date of filing: 20.05.2008
(51) Int. Cl.: A61L 2/10, A61L 2/20

(54) **Method for desinfection and sterilization**

(71) Applicant: Schaumburg, Christiane, 2930 Klampenborg (DK)
(72) Inventor: Schaumburg, Christiane, 2930 Klampenborg (DK)
(74) Representative: Benthin, Stig

(57) **Abstract**

The present invention relates to a method for sterilization or disinfection of an object or a composition using a water soluble precursor for a highly reactive species and UV radiation. The method comprising contacting of said object or composition with a water soluble precursor for a highly reactive species in the presence of water, and irradiating said object or composition with UV radiation having a peak wave length and intensity allowing the in-situ conversion of said at least one precursor into a highly reactive species. The method generates in-situ the highly reactive species from a combination of one or more precursors under the influence of electromagnetic radiation. The radiation is focused on the region to be disinfected or sterilized and generates from the precursors the highly reactive species. The precursors are preferably selected from non-toxic environmental benign compounds. Only under irradiation the one or more precursors give rise to the creation of the highly reactive species. The reactive species have a short lifetime reverting to stable benign compound. Examples of highly reactive species are hydroxyl radical, NO radical, ozonide and CIO.

## Description

### Field of the invention

The present invention relates to the field of sterilization and desinfection of objects and compositions.

### Background of the invention

Within the health care system as well as within a number of other industries, such as semiconductors, aeronautics etc, there are requirements for disinfecting or sterilizing a number of objects and compositions which cannot withstand high temperatures. A number of well established techniques are available which may be used for disinfecting or sterilizing such objects and compositions.

Glutaraldehyde has been successfully used for disinfecting and sterilizing medical instruments. Glutaraldehyde is very reactive and thus has an effective antimicrobial action. Glutaraldehyde is an irritant and sensitizer, with inconsistent data on toxicity, teratogenicity and carcinogenicity. However, it is well documented that the common use of glutaraldehyde within the health care system has lead to occupationally related illnesses of staff that operate the equipment using glutaraldehyde.

Sterilization by ethylene oxide has many of the same inherent problems as those of glutaraldehyde. Thus, ethylene oxide is not a solution for use in decentralized health care systems.

Ultraviolet (UV) light has also been used successfully in, e.g. the health care system, for the sterilization and disinfection of medical instruments and fluids. UV light is a spectrum of light just below the range visible to the human eye (below the blue spectrum of visible light). UV light is divided into four distinct spectral areas : Vacuum UV (100 to 200 nm), UV-C (200 to 280 nm), UV-B (280 to 315 nm), and UV-A (315 to 400 nm). The UV-C spectrum from 200 nm to 280 nm is the most lethal range of wavelengths for microorganisms, the peak germicidal wawelength being 264 nm. Sterilization and desinfection of medical instruments using UV light with a maximum intensity within the wavelength of approx. 250 to 270 nm are commonly used. UV light having this wavelength is known to impair DNA by intrastrand linkage of adjacent pyrimidines, usually thymines, called thymine dimers, thus being either lethal to a cell or at least preventing the proliferation of the cell. The use of UV light for sterilization has the disadvantage that many microorganisms have available a number of repair-systems directed to the repair of UV induced damage to their DNA. Also, a significant dose of UV radiation is neccesary to cause a desinfiction or sterilization, and the UV radiation must reach all of the contaminating microorganisms.

An example of sterilization at the germicidal wavelength is disclosed in US 2005/0079096 A1. A method for sterilizing a medical device comprising the the step of exposing said medical device to monochromatic UV radiation knowing the Dᵥₐₗᵤₑ of spores of *Bacillus Stearothermophilus* (ATCC 7953) is at least 23.7 mJ/cm2 monochromatic UV radiation at 257 nm.

Sterilization by UV radiation alone has the disadvantage that if any part of the object or liquid to be sterilized is not exposed to the UV radiation then the sterilization fails. For many objects and liquids all parts are not readily available for the UV radiation.

Medical instruments amenable for sterilization have been disclosed where the instrument is covered by a TiO₂ photocatalytic film by low-pressure metal-organic chemical vapor deposition (Byong-Hoon et al., Biotechnol. Bioproc. Eng. 12 (2007) 136-139). It was concluded that survival of Streptococcus mutans was 0.01 % when attached onto TiO₂ film and exposed to UV-A radiation for 15 minutes. The method is not generally applicable for reduction of microbial load as it only relates to solid surfaces and it requires that the entire surface is coated with a TiO₂ film and exposed to radiation.

A synergistic antibacterial effect between blue light having a wave length in the range from 400 nm to 500 nm and H₂O₂ has been observed by Feuerstein et al., J. Antimicrob. Chemoth. 57 (2006) 872-876.

Other methods for sterilization have been used which are dependent on specialised equipment and facilities meeting the required safety levels, and thus they are only applicable for centralised, industrial sterilization. Examples of such methods are e.g. gamma and electron radiation.

Formulations of ozonides have been used as antimicrobial/antiparasite formulas in the dental industry. However, due to their explosive nature the use of ozonides for the preparation of such formulations are hazardous.

### Summary of the invention

In order to overcome the above-mentioned limitations of the known methods for desinfecting or sterilizing an object or a composition, the present invention provides a method for chemically desinfecting or sterilizing an object or a composition, said method comprising the steps :
- contacting of said object or composition with a water soluble precursor for a highly reactive species in the presence of water, and
- irradiating said object or composition with UV radiation having a peak wave length and intensity allowing the *in-situ* conversion of
   said at least one precursor into a highly reactive species.

The method generates in-situ the highly reactive species from a combination of one or more precursors under the influence of electromagnetic radiation. The radiation is focussed on the region to be disinfected or sterilized and generates from the precursors the highly reactive species. The precursors are preferably selected from non-toxic environmental benign compounds. Only under irradiation the one or more precursors give rise to the creation of the highly reactive species. The reactive species have a short lifetime reverting to stable benign compounds

In another aspect the present invention provides the use of the above mentioned method for handwashing prior to surgery, disinfection of skin prior to surgery, disinfection or sterilization of a surgery, facilities for birds such as facilities for chicken or turkeys, reduction of *Salmonella*, facilities for animal testing, cow teats prior to milking, horticulture, slautherhouses, fishing industry, vacuum packaging facilities, clean rooms, or reducing the microbial load prior to other sterilization e.g. radiation or gas sterilization.

### Detailed description of the invention

The present invention provides a method for chemically desinfecting or sterilizing an object or a composition, said method comprising the steps :
- contacting of said object or composition with a water soluble precursor for a highly reactive species in the presence of water, and
- irradiating said object or composition with UV radiation having a peak wave length and intensity allowing the *in-situ* conversion of
   said at least one precursor into a highly reactive species.

Thus the method for chemically desinfecting or sterilizing an object or a composition, said method comprising the contacting of said object or composition with a water soluble precursor for a highly reactive species in the presence of water, and irradiating said object or composition with UV radiation having a peak wave length and intensity allowing the *in-situ* conversion of said precursor into a highly reactive species. In one embodiment the UV irradiation is performed simulataneously with said object or composition being in contact with said water soluble precursor for a highly reactive species in the presence of water.

UV light sources primarily come as low-pressure or medium/high-pressure lamps. Low-pressure lamps produce virtually all of their UV output at a wavelength of 254 nm, which is very close to the peak germicidal effectiveness curve. These lamps generally convert up to 40% of their input watts into UV-C watts. This is much higher than other classes of lamps. Low-pressure lamps typically run on low-input power currents and they have a useful life of 8,000 to 12,000 hours depending on the operating current of the lamp.

Medium/high-pressure lamps produce wavelengths widely ranging from 100 nm to greater than 700 nm, well into the visible light spectrum. These lamps are very poor producers of UV-C wavelengths; they generally convert only up to 7% of their input watts into UV-C watts. The remaining watts are converted into heat and visible light. Medium/high-pressure lamps typically run on high-input power currents and have a useful life of only 1,000 to 2,000 hours depending on the lamp's operating current. As is evident, low-pressure lamps have a much better efficiency as compared to the medium/highpressure lamps.

UV light emitting diodes (UV LED's) have also become commercialle available, e.g. from Fox Group, Ocean Optics, Nichia and Roithner Lasertechnik. UV LED's produce UV radiation having a very narrow wavelength versus intensity profile, and most of the input poweris converted into the desired UV radiation. UV-LEDs's come in a wide variety of wavelength from 255 nm to 400 nm in steps of 5 nm, and their small physical dimension as well as their small energy requirements makes them well suited for small devices for sterilizaing or disinfecting objects and composition. One example of UV-LED's is NSHU550A (from Nichia) emitting UV-A at 375 nm with a spectrum half-width of 10 nm and a power dissipation of 100 mW. Another example is UVTOP255-FW-TO39 (from Roithner Lasertechnik) emmitting UV-C radiation having a wavelength of 255 nm with a spectrum half-width of 12 nm and a power dissipation of 150 mW. The diodes are operated by low voltage DC power permitting construction of small and portable units.

The UV radiation may be provided using any convenient source, e.g. a lamp, a laser or a LED diode.

The term "approximately" or "approx." as used herein in relation to a wavelength of UV radiation is intended to mean about the specified wave length +/- 25 nm. Thus, "approx. 230 nm" means from 205nm to 255 nm, such as from 210 nm to 250 nm, such as from 215 nm to 245 nm, such as from 225 nm to 235 nm.

In one embodiment the UV radiation has a peak wave length of approx. 190 nm.

In another embodiment the UV radiation has maximum intensity at a wave length of less than 260 nm, at less than 240 nm, or at less than 200 nm.

In another embodiment the intensity of the UV radiation is in the range from 1 J/cm² to 100 J/cm². In another embodiment the intensity of the UV radiation is in the range from 0.1 J/cm² to 50 J/cm². In another embodiment the intensity of the UV radiation is in the range from 10 J/cm² to 1000 J/cm². In another embodiment the intensity of the UV radiation is in the range from 0.01 J/cm² to 1 J/cm².

In another embodiment the UV radiation is applied for a period in the range from about 0.1 s to about 120 minutes, such as from about 30 s to about 45 minutes, such as from about 2 minutes to about 10 minutes.

A sterilization method is considered to reduce the microbial load by a factor 10⁶. A desinfection method is considered to reduce microbial load by a factor of 10⁵. Representative spore preparations, e.g. *Bacillus stearothermophilus* (ATCC 7953) spores, for testing in sterilization and disinfection methods are commercially available, e.g. from Sigma-Aldrich.

The term "highly reactive species" as used herein means one or more chemical entities having a very short half-life once formed due to their high reactivity with other compounds. Non-limiting examples of highly reactive species are hydroxyl radical, NO radical, ozonide and CIO.

Ozonide is an unstable, reactive polyatomic anion O³⁻, derived from ozone, or an organic compound similar to organic peroxide formed by a reaction of ozone with an unsaturated compound.

Inorganic ozonides are dark red ionic compounds containing the reactive O³⁻ anion. The anion has the V shape of the ozone molecule. Inorganic ozonides are formed by burning sodium or heavier alkali metals in ozone. They are very sensitive explosives that have to be handled at low temperatures in an atmosphere comprised of an inert gas. Evidently, inorganic ozonides are not suitable as antimicrobial agents as they cannot be generated in-situ and as they are hazardous compounds which are not easily prepared and formulated.

Organic ozonides are more explosive relatives of the organic peroxides and contain a covalently bonded ozonide group, -O-O-O-. They usually appear in the form of foul-smelling oily liquids. Their main use is in determining the structure of chemical compounds. As intermediates of ozonolysis, they are formed by an addition reaction of ozone and unsaturated compounds, and rapidly decompose to carbonyl compounds, aldehydes, ketones or peroxides. Organic ozonides are formed as 1,2,4-trioxolanes by the reaction of ozone at a carbon-carbon double bond, or the analogous compounds derived from acetylenic compounds.

In one embodiment the highly reactive species has a half-life of less than 1 second at the conditions prevailing during the desinfection or sterilization. In another embodiment the highly reactive species has a half-life of less than 100 ms, less than 1 ms or less than 0.1 ms at the conditions prevailing during the desinfection or sterilization.

In another embodiment the precursor is oxygen and said highly reactive species is ozonide.

In another embodiment the precursor is an alkene.

In another embodiment the precursor is a dioxide precursor and the UV radiation has a peak wave length of approx. 230 nm.

In yet another embodiment the precursor is a peroxide precursor and the UV radiation has a peak wave length of approx. 230 nm.

In another embodiment the reactive species is CIO as produced by UV-C radiation.

In another embodiment the reactive species is a NO radical as produced by UV-C radiation.

In another embodiment the precursor is hexachloroplatinate generating aminochloroplatinate by UV radiation at approx 254 nm.

In another embodiment the reactive species is an ozonide generated by UV-C radiation of an alkene derived from a plant.

In another embodiment the object or composition is placed in a sealed container during the sterilization or disinfection.

In another embodiment the object or composition is a fluid, such as a suspension or a solution, e.g. an aqueous solution.

In another embodiment the object or composition is a medical device, such as surgical instruments, implantable devices, electronic equipment for testing or diagnosing a patient.

In another embodiment the object or composition is one or more contact lenses.

In another aspect the present invention provides the use of the method for handwashing prior to surgery, disinfection of skin prior to surgery, disinfection or sterilization of a surgery, facilities for brids such as facilities for chicken or turkeys, reduction of *Salmonella*, facilities for animal testing, cow teats prior to milking, horticulture, sloutherhouses, fishing industry, vacuum packaging facilities, clean rooms, or reducing the microbial load prior to other sterilization e.g. radiation or gas sterilization.

### EXAMPLES

### EXAMPLE 1.

Microparticles of anastas or doped anastas are dispersed in the water applied to surface to be cleaned. UV radiation in the UV-A range is applied. Hereby ozonide and hydroxyl radicals are formed and disinfection is initiated. The necessary exposure time is determined as the time sufficient to ensure that disinfection is achieved.

### EXAMPLE 2.

Microparticles consisting of metal nanoparticles dispersed in aerogel or xerogel particles where the gel contains Ti and Si oxides are suspended in the water applied to the surface to be cleaned. UV radiation in the UV- range is applied to generate ozonide and hydroxyl radicals. The necessary exposure time is determined as the time sufficient to ensure that disinfection is achieved. Sonication can be used to increase the rate of disinfection or sterilization.

### EXAMPLE 3.

Microparticles containing perborates are dispersed in the water applied to the surface to be cleaned. UV radiation in the UV- range is applied to generate ozonide and hydroxyl radicals. The necessary exposure time is determined as the time sufficient to ensure that disinfection is achieved. Sonication can be used to increase the rate of disinfection or sterilization.

### EXAMPLE 4.

Microparticles containing percarbonates are dispersed in the water applied to the surface to be cleaned. UV radiation in the UV- range is applied to generate ozonide and hydroxyl radicals. The necessary exposure time is determined as the time sufficient to ensure that disinfection is achieved. Sonication can be used to increase the rate of disinfection or sterilization.

### EXAMPLE 5.

Organic ozonides are formed as 1,2,4-trioxolanes by the reaction of ozone at a carbon-carbon double bond are incorporated in hydrogels to a concentration of 1-10% applying usual procedures. The hydrogel may be used directly or it may be converted to an aerogel by freeze drying or in supercritical CO2. By dilution in the gel the ozonide is stabilized and its smell strongly reduced. The gel powder is dispersed in the water applied to the surface to be cleaned. Sonication is used to increase the release of ozonide and hydroxyl radicals from the gel and disinfection is initiated. The sufficient exposure time for ensuring disinfection or sterilization is determined.

## Claims

1. A method of chemically desinfecting or sterilizing an object or a composition, said method comprising the steps :
- contacting of said object or composition with a water soluble precursor for a highly reactive species in the presence of water, and
- irradiating said object or composition with UV radiation having a peak wave length and intensity allowing the *in-situ* conversion of said at least one precursor into a highly reactive species.

2. The method according to any one of the preceding claims, wherein said UV radiation is provided by a light emitting diode (LED).

3. The method according to any one of the preceding claims, wherein said highly reactive species has a half-life of less than 1 second at the conditions prevailing during the desinfection or sterilization.

4. The method according to any one of the preceding claims, wherein said precursor is oxygen and said highly reactive species is ozonide.

5. The method according to claim 3, wherein said precursor is an alkene.

6. The method according to any one of claims 4-5, wherein said UV radiation has a peak wave length of approx. 190 nm.

7. The method according to any one of claims 1-3, wherein said precursor is a dioxide precursor and said UV radiation has a peak wave length of approx. 230 nm.

8. The method according to any one of claims 1-3, wherein said precursor is a peroxide precursor and said UV radiation has a peak wave length of approx. 230 nm.

9. The method according to any one of claims 1-3 and 5, wherein said reactive species is an ozonide generated by UV-C radiation of an alkene derived from a plant.

10. The method according to any one of the preceding claims, wherein said object or composition is placed in a sealed container during the sterilization or disinfection.

11. The method according to any one of the preceding claims, wherein said object or composition is a fluid, such as a suspension or a solution, e.g. an aqueous solution.

12. The method according to any one of the preceding claims, wherein said object or composition is a medical device.

13. The method according to any one of the preceding claims, wherein said object or composition is one or more contact lenses.

14. Use of the method according to any of the preceding claims for handwashing prior to surgery, disinfection of skin prior to surgery, disinfection or sterilization of a surgery, facilities for brids such as facilities for chicken or turkeys, reduction of *Salmonella*, facilities for animal testing, cow teats prior to milking, horticulture, sloutherhouses, fishing industry, vacuum packaging facilities, clean rooms, or reducing the microbial load prior to other sterilization e.g. radiation or gas sterilization.
